(19) Europäisches Patentamt
European Patent Office
Office européen des brevets

(11) Veröffentlichungsnummer: **0 337 255 A1**

(12) **EUROPÄISCHE PATENTANMELDUNG**

(21) Anmeldenummer: **89105916.4**

(51) Int. Cl.4: **C07D 233/92 , C07B 43/04**

(22) Anmeldetag: **05.04.89**

(30) Priorität: **14.04.88 DE 3812401**

(43) Veröffentlichungstag der Anmeldung:
**18.10.89 Patentblatt 89/42**

(84) Benannte Vertragsstaaten:
**CH DE FR GB IT LI NL**

(71) Anmelder: **BASF Aktiengesellschaft**
**Carl-Bosch-Strasse 38**
**D-6700 Ludwigshafen(DE)**

(72) Erfinder: **Koehler, Hermann, Dr.**
**Roentgenstrasse 7**
**D-6711 Beindersheim(DE)**
Erfinder: **Dockner, Toni, Dr.**
**Grossgasse 6**
**D-6701 Meckenheim(DE)**

(54) **Verfahren zur Herstellung von 4-Nitro-5-aminoimidazolen.**

(57) Verfahren zur Herstellung von 4-Nitro-5-aminoimidazolen der Formel I

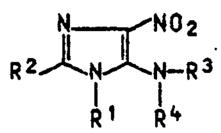

(I),

in der
$R^1$ einen organischen Rest,
$R^2$ Wasserstoff oder einen organischen Rest und
$R^3$, $R^4$ voneinander unabhängig oder gemeinsam einen organischen Rest bedeuten, wobei die organischen Reste noch unter den Reaktionsbedingungen inerte Substituenten tragen können, dadurch gekennzeichnet, daß man Dinitroimidazole der Formel II

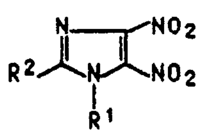

(II)

mit einem Amin der Formel III

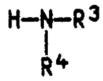

(III),

in der $R^3$ und $R^4$ die obengenannten Bedeutungen haben, in einem Lösungs-oder Verdünnungsmittel bei einem pH-Wert von 4 bis 14 umsetzt.

EP 0 337 255 A1

## Verfahren zur Herstellung von 4-Nitro-5-aminoimidazolen

Die vorliegende Erfindung betrifft ein Verfahren zur Herstellung von 4-Nitro-5-aminoimidazolen.

Aus J. Prakt. Chem. 22,304-313 (1963) und Acta Pol. Pharm. 41, 397-400 (1984) ist bekannt, daß die Verbindungen der Formel I mit $R^1 = CH_3$ und $R^2$ = H durch Umsetzung von 1-Methyl-4-nitro-5-chlorimidazol mit Aminen erhalten werden.

Das 1-Methyl-4-nitro-5-chlorimidazol ist nur in schlechter Ausbeute durch Umsetzung von N,N′-Dimethyloxalsäurediamid mit Phosphorpentachlorid und nachfolgende Nitrierung des gebildeten 1-Methyl-5-chlorimidazols zugänglich (O. Wallach, Liebigs Annalen der Chemie 184, 51 (1877)).

Ein weiterer Nachteil des vorbeschriebenen Verfahrens ist die geringe Anwendungsbreite.

Der Erfindung lag daher die Aufgabe zugrunde, ein neues und verbessertes Verfahren zur Herstellung von 4-Nitro-5-aminoimidazolen zu finden und den Nachteilen des bekannten Verfahrens abzuhelfen.

Demgemäß wurde ein neues und verbessertes Verfahren zur Herstellung von 4-Nitro-5-aminoimidazolen der allgemeinen Formel I

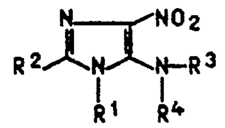

(I),

in der

$R^1$ einen organischen Rest,

$R^2$ Wasserstoff oder einen organischen Rest und $R^3$, $R^4$ voneinander unabhängig oder gemeinsam einen organischen Rest bedeuten, wobei die organischen Reste noch unter den Reaktionsbedingungen inerte Substituenten tragen können, gefunden, welches dadurch gekennzeichnet ist, daß man sie vorteilhaft erhält, wenn man Dinitroimidazole der Formel II

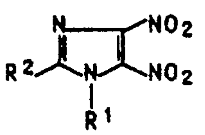

(II)

mit einem Amin der Formel III

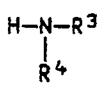

(III),

in der $R^3$ und $R^4$ die vorgenannten Bedeutungen haben, in einem Lösungs-oder Verdünnungsmittel bei einem pH-Wert von 4 bis 14 umsetzt.

Der Erfolg des erfindungsgemäßen Verfahrens ist überraschend insbesondere im Hinblick darauf, daß selektiv nur die Nitrogruppe in 5-Stellung des Imidazols II ausgetauscht wird.

Die Herstellung von Verbindungen der Formel II ist aus Chem. Heterocyclic Compds. 1970, 465 (vgl. CA 73, 66491 z) bekannt. Nach dem dort angegebenen Verfahren ist eine große Zahl von verschieden substituierten Verbindungen der Formel II zugänglich.

Der organische Rest $R^1$ bedeutet beispielsweise Alkyl, Alkoxyalkyl, Cycloalkyl, Aryl, Aralkyl oder Alkylaryl. Alkylreste sind z.B. verzweigte oder unverzweigte Reste mit 1 bis 18, insbesondere 1 bis 8 Kohlenstoffatomen wie Methyl, Ethyl, Propyl, Isopropyl, n-Butyl, iso-Butyl, sec.-Butyl, tert.-Butyl, Pentyl, Hexyl, Heptyl oder Octyl. Alkoxyalkylreste haben z.B. insgesamt 2 bis 8 Kohlenstoffatome wie $C_1$- bis $C_4$-Alkoxymethyl, Methoxyethyl oder Ethoxyethyl. Cycloalkylreste sind z.B. $C_5$- bis $C_8$-Cycloalkylreste, insbesondere Cyclopentyl oder Cyclohexyl. Arylreste sind z.B. Phenyl, Naphthyl, ggf. substituiert durch inerte Gruppen wie $C_1$- bis $C_4$-Alkyl, $C_1$- bis $C_4$-Alkoxy, Halogen wie Fluor, Chlor oder Brom, Halogenalkyl wie Trifluormethyl, Difluormethyl oder Dichlormethyl oder $C_1$- bis $C_4$-Alkoxymethyl. Aralkyl- oder Alkylarylreste sind insbesondere solche mit 7 bis 12 Kohlenstoffatomen wie Tolyl, Xylyl, Phenethyl oder Benzyl, die

ebenso wie die Arylreste substituiert sein können.

Der Rest $R^2$ kann die Bedeutung von $R^1$ haben und darüber hinaus für Wasserstoff stehen. Die organischen Reste $R^1$ und/oder $R^2$ können noch unter den Reaktionsbedingungen inerte Substituenten tragen wie $C_1$- bis $C_4$-Alkyl- oder Alkoxygruppen oder Halogen wie Fluor, Chlor oder Brom. Der Rest $R^2$ steht bevorzugt für Wasserstoff, $C_1$-$C_4$-Alkyl wie Methyl, Ethyl, Propyl, Isopropyl oder Butyl, Alkoxymethyl wie Methoxymethyl; für Phenyl; Alkylaryl wie p-Tolyl und Aralkyl wie Benzyl und Phenethyl.

Die Amine der Formel III sind bekannt und können nach literaturüblichen Verfahren hergestellt werden.

Die organischen Reste $R^3$ und $R^4$ bedeuten unabhängig voneinander beispielsweise Wasserstoff, Alkyl, Alkoxyalkyl, Cycloalkyl, Aryl, Arylalkyl oder Alkylaryl, d.h. die für $R^1$ genannte Bedeutung, wobei die genannten Reste noch durch inerte Gruppen wie $C_1$- bis $C_4$-Alkyl, $C_1$- bis $C_4$-Halogenalkyl, $C_1$- bis $C_4$-Alkoxy, $C_1$- bis $C_4$-Alkylthio oder durch Halogen wie Fluor, Chlor oder Brom substituiert sein können. $R^3$ und/oder $R^4$ steht z.B. für Wasserstoff, $C_1$- bis $C_4$-Alkyl wie Methyl, Ethyl, Propyl, Isopropyl oder Butyl, Aryl oder Aralkyl wie Phenyl, 1-Naphthyl, 2-Naphthyl, 4-Chlorphenyl, 2,4-Dichlorphenyl, 2-Bromphenyl, 4-Methoxyphenyl, 3-(Trifluormethyl)-phenyl, 3-(Difluormethyl)-phenyl, Benzyl oder 4-Chlorbenzyl.

Weiterhin können $R^3$ und $R^4$ gemeinsam mit dem Stickstoffatom einen heterocyclischen oder heteroaromatischen Rest, der gegebenenfalls durch $C_1$- bis $C_4$-Alkyl, $C_1$- bis $C_4$-Alkoxy oder Halogen substituiert ist, bedeuten, wobei als Heteroatome insbesondere Stickstoff neben Sauerstoff und Schwefel in Betracht kommen. Die heteroaromatischen Reste haben vorteilhaft 5 oder 6 Ringglieder mit 1 bis 2 Heteroatomen und können einen weiteren 5- oder 6-Ring, der z.B. aromatisch heteroaromatisch ist, ankondensiert haben. Beispielsweise seien folgende Reste aufgeführt: N-Pyrrolyl, N-Imidazolyl, N-Pyrazolyl, N-Triazolyl oder N-Tetrazoyl. Heterocyclische Reste sind z.B. gesättigte oder Doppelbindungen enthaltende heterocyclische Reste mit 1 oder 2 Heteroatomen wie Pyrrolidinyl, Piperidinyl oder Morpholinyl.

Nach dem erfindungsgemäßen Verfahren können 4-Nitro-5-aminoimidazole der Struktur I in einfacher Weise dadurch erhalten werden, daß man Dinitroimidazole II mit den Verbindungen III in einem Lösungs- oder Verdünnungsmittel bei einem pH-Wert von 4 oder darüber, z.B. 4 bis 14, insbesondere 6 bis 14, besonders vorteilhaft 8 bis 13, miteinander umsetzt.

Als Lösungs- oder Verdünnungsmittel kommen insbesondere Wasser und/oder mit Wasser mischbare inerte organische Lösungsmittel in Betracht. Geeignete organische Lösungsmittel sind z.B. niedermolekulare Alkohole, Glykole und Glykolderivate wie Ethylenglykol, Methyl- oder Ethylglykol, Ether wie Tetrahydrofuran oder Dioxan sowie Säureamide wie N,N-Dimethylformamid, N-Methylpyrrolidon oder N,N,N′,N′-Tetramethylharnstoff. Bevorzugte Lösungsmittel sind Wasser und/oder $C_1$-$C_4$-Alkohole, z.B. Methanol, Ethanol, Propanol, Isopropanol, Butanol und Isobutanol.

Der pH-Wert des Reaktionsgemisches wird im allgemeinen durch Zugabe einer Base eingestellt. Im Falle der Umsetzung mit Ammoniak oder einfachen primären und sekundären Aminen wie Methylamin, Dimethylamin oder Diethylamin kann man den pH-Wert durch einen Überschuß des eingesetzten Amins einstellen. Ferner können organische Basen wie Trimethylamin oder Triethylamin oder anorganische Basen, beispielsweise Alkali- oder Erdalkalimetallhydride wie Lithiumhydrid, Natriumhydrid, Kaliumhydrid oder Calciumhydrid, bevorzugt in aprotisch dipolaren Lösungsmitteln, eingesetzt werden.

Gegebenenfalls können die Amine auch als Alkali- oder Erdalkalimetallsalze in die Reaktion eingesetzt werden. Beispiele für diese Amine sind Pyrazole oder Imidazole, deren Alkali- oder Erdalkalimetallsalze vorteilhaft durch Umsetzung der Verbindungen mit Basen wie Alkali- oder Erdalkalimetallalkoholaten oder Alkali- oder Erdalkalimetallhydriden erhalten werden.

Die Basenmenge ist nicht besonders kritisch, es können z.B. 0,1 bis 100 mol, vorteilhaft 1 bis 20 mol, besonders vorteilhaft 1 bis 3 mol je Mol Dinitroimidazol II verwendet werden.

Das Molverhältnis der Verbindung III, bezogen auf das Dinitroimidazol II beträgt zweckmäßigerweise 1 bis 5, vorteilhaft 1 bis 3, besonders vorteilhaft 1 bis 1,2.

Die Reaktion kann unter äußerst milden Bedingungen durchgeführt werden. Temperaturen von 0 bis 150 °C sind ausreichend. Bevorzugt wird die Umsetzung bei Temperaturen von 20 bis 80 °C vorgenommen.

Die Reaktion wird üblicherweise bei Normaldruck kontinuierlich oder diskontinuierlich in den dafür geeigneten Apparaturen durchgeführt. Im Falle von flüchtigen Verbindungen III, kann es vorteilhaft sein, unter leichtem Überdruck in einer geschlossenen Apparatur zu arbeiten.

Die Isolierung und Reinigung der Produkte erfolgt in an sich bekannter Weise z.B. durch Abfiltrieren der festen Produkte und Umkristallisieren aus geeigneten Lösungsmitteln.

Nach der erfindungsgemäßen Verfahrensweise fallen die 4-Nitro-5-aminoimidazole in guten bis sehr guten Ausbeuten an.

4-Nitro-5-aminoimidazole I sind als Zwischenprodukte zur Herstellung von Farbstoffen, Pflanzenschutzmitteln und Pharmazeutika von Interesse.

Die folgenden Beispiele sollen die Erfindung näher erläutern, ohne sie einzuschränken.

Beispiele

Beispiel 1

Herstellung von 1-Methyl-4-nitro-5-aminoimidazol

17,2 g (0,1 mol) 1-Methyl-4,5-dinitroimidazol wurden bei 30°C unter Rühren in 200 ml 25 %iges wäßriges Ammoniak eingetragen. Nach 2 Stunden wurde das ausgefallene Produkt abgesaugt und mit Wasser neutral gewaschen. Man erhielt 13,5 g (95 %) 1-Methyl-4-nitro-5-aminoimidazol; Fp. 309°C (Zers.).

Entsprechend Beispiel 1 wurden die in Tabelle 1 aufgeführten Beispiele durchgeführt.

Tabelle 1

| Beisp. | $R^1$ | $R^2$ | $R^3$ | $R^4$ | Ausbeute | Smp. (°C) | Elementaranalyse | | |
|---|---|---|---|---|---|---|---|---|---|
| 2 | $CH_3$ | H | $CH_3$ | H | 45 % | 155 - 157 | | | |
| 3 | $CH_3$ | H | $CH_3$ | $CH_3$ | 51 % | 94 - 95 | | | |
| 4 | Benzyl | H | $CH_3$ | $CH_3$ | 54 % | 71 - 73 | Ber.: | C 58,5; N 22,8 | H 5,7; |
| | | | | | | | Gef.: | C 58,2; N 22,6 | H 5,4; |

Beispiel 5

Herstellung von 1-Methyl-4-nitro-5-piperidinoimidazol

17,2 g (0,1 mol) 1-Methyl-4,5-dinitroimidazol wurden in 100 ml Ethanol gelöst, 17,0 g (0,2 mol) Piperidin zugegeben und die Mischung 1 Stunde zum Sieden erhitzt. Nach Abkühlung wurde der ausgefallene Niederschlag abgesaugt und aus Ethanol umkristallisiert. Man erhielt 13,9 g (66 %) 1-Methyl-4-nitro-5-piperidinoimidazol, Fp. 175 - 176°C.

| | | | |
|---|---|---|---|
| Ber.: | C 51,4; | H 6,7; | N 26,7. |
| Gef.: | C 51,4; | H 6,2; | N 26,8. |

Beispiel 6

Herstellung von 1,2-Dimethyl-4-nitro-5-piperidinoimidazol

Entsprechend Beispiel 5 erhielt man durch Umsetzung von 1,2-Dimethyl-4,5-dinitroimidazol mit Piperidin das 1,2-Dimethyl-4-nitro-5-piperidinoimidazol, Fp. 170 - 172°C in einer Ausbeute von 58 %.

| | | | |
|---|---|---|---|
| Ber.: | C 53,6; | H 7,2; | N 25,0. |
| Gef.: | C 53,7; | H 6,6; | N 25,1. |

Beispiel 7

Herstellung von 1-Methyl-4-nitro-5-anilinoimidazol

17,2 g (0,1 mol) 1-Methyl-4,5-dinitroimidazol wurden in 100 ml Ethanol gelöst, 11,2 g (0,12 mol) Anilin und 12,1 g (0,12 mol) Triethylamin zugegeben und die Mischung 6 Stunden bei 60° C gerührt. Nach Abkühlung wurde der ausgefallene Niederschlag abgesaugt und aus Ethanol umkristallisiert. Man erhielt 9,4 g (43 %) 1-Methyl-4-nitro-5-anilinoimidazol; Fp. 169 - 170° C.

| | | | |
|---|---|---|---|
| Ber.: | C 55,0; | H 4,8; | N 25,7. |
| Gef.: | C 55,0; | H 4,6; | N 25,8. |

Beispiel 8

Herstellung von 1-Methyl-4-nitro-5-(4-dimethylamino)-anilinoimidazol

Entsprechend Beispiel 7 erhielt man durch Umsetzung von 1-Methyl-4,5-dinitroimidazol mit (4-Dimethylamino)-anilin das 1-Methyl-4-nitro-5-(4-dimethylamino)-anilinoimidazol; Fp. 195° C (Zers.) in einer Ausbeute von 58 %.

| | | | |
|---|---|---|---|
| Ber.: | C 55,2; | H 5,8; | N 26,8. |
| Gef.: | C 55,2; | H 5,9; | N 26,7. |

Beispiel 9

Herstellung von 1-Methyl-4-nitro-5-pyrazoloimidazol

8,2 g (0,12 mol) Pyrazol wurden in 100 ml Dimethylformamid gelöst, 22 g (0,12 mol) 30 %iger Natriummethylatlösung in Methanol zugegeben und 50 ml Methanol/DMF-Gemisch abdestilliert. Zum Rückstand wurden 17,2 g (0,1 mol) 1-Methyl-4,5-dinitroimidazol gegeben und anschließend 30 Minuten bei 50° C gerührt. Nach Abkühlung wurde das ausgefallene Salz abfiltriert und das Filtrat eingeengt. Der Rückstand wurde mit Isopropanol in der Hitze gelöst und anschließend durch Zugabe von Ether gefällt. Man erhielt 15,6 g (81 %) 1-Methyl-4-nitro-5-pyrazoloimidazol, Fp. 126 - 127° C.

| | | | |
|---|---|---|---|
| Ber.: | C 43,5; | H 3,7; | N 36,3. |
| Gef.: | C 43,0; | H 3,9; | N 35,2. |

Entsprechend Beispiel 9 wurden die in Tabelle 2 aufgeführten Beispiele durchgeführt.

Tabelle 2

| Beisp. | $R^1$ | $R^2$ | III | Ausbeute | Smp. (°C) | Elementaranalyse | | |
|---|---|---|---|---|---|---|---|---|
| 10 | $CH_3$ | H | Imidazol | 74 % | 158 - 160 | | | |
| 11 | $CH_3$ | H | 4,5-Dichlorimidazol | 86 % | 230 - 231 | Ber.: | C 32,1; | H 1,9; |
| | | | | | | | N 26,7; | Cl 27,1 |
| | | | | | | Gef.: | C 32,8; | H 2,1; |
| | | | | | | | N 25,2; | Cl 27,1 |
| 12 | $CH_3$ | $CH_3$ | 4,5-Dichlorimidazol | 71 % | 215 - 216 | Ber.: | C 34,8; | H 2,6; |
| | | | | | | | N 25,4; | Cl 25,7 |
| | | | | | | Gef.: | C 34,9; | H 2,7; |
| | | | | | | | N 25,4; | Cl 25,5 |

**Ansprüche**

1. Verfahren zur Herstellung von 4-Nitro-5-aminoimidazolen der Formel I

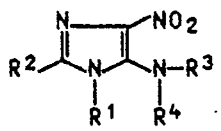

(I),

in der
$R^1$ einen organischen Rest,
$R^2$ Wasserstoff oder einen organischen Rest und
$R^3$, $R^4$ voneinander unabhängig oder gemeinsam einen organischen Rest bedeuten, wobei die organischen Reste noch unter den Reaktionsbedingungen inerte Substituenten tragen können, dadurch gekennzeichnet, daß man Dinitroimidazole der Formel II

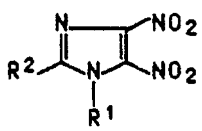

(II)

mit einem Amin der Formel III

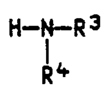

(III),

in der $R^3$ und $R^4$ die genannten Bedeutungen haben, in einem Lösungs-oder Verdünnungsmittel bei einem pH-Wert von 4 bis 14 umsetzt.

2. Verfahren nach Anspruch 1, dadurch gekennzeichnet, daß man als Lösungs- oder Verdünnungsmittel Wasser und/oder mit Wasser mischbare inerte organische Lösungsmittel verwendet.

3. Verfahren nach den Ansprüchen 1 und 2, dadurch gekennzeichnet, daß man als Lösungs- oder Verdünnungsmittel Wasser und/oder $C_1$-$C_4$-Alkohole verwendet.

4. Verfahren nach den Ansprüchen 1 bis 3, dadurch gekennzeichnet, daß man die Umsetzung bei einem pH-Wert von 6 bis 14 durchführt.

5. Verfahren nach den Ansprüchen 1 bis 4, dadurch gekennzeichnet, daß man den pH-Wert durch Zusatz einer Base einstellt.

6. Verfahren nach den Ansprüchen 1 bis 5, dadurch gekennzeichnet, daß man pro Mol Dinitroimidazol II 1 bis 20 mol Base verwendet.

7. Verfahren nach den Ansprüchen 1 bis 6, dadurch gekennzeichnet, daß man pro Mol Dinitroimidazol II 1 bis 5 mol der Verbindung III verwendet.

Europäisches Patentamt

# EUROPÄISCHER RECHERCHENBERICHT

Nummer der Anmeldung

EP 89 10 5916

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | SOVIET INVENTIONS ILLUSTRATED Derwent Publications Ltd., Sektion Chemie, Woche 8409, Zusammenfassung Nr. 054380, E13, 1. April 84;& SU - A - 1011643 (KAZAN KIROV CHEMTECH) 15.04.1983; & CHEMICAL ABSTRACTS, Band 9, Nr. 9, 29. August 1983; Columbus, Ohio, US; Seite 630, Spalte 1, Zusammenfassung Nr. 70731g<br>--- | 1-7 | C 07 D 233/92<br>C 07 B 43/04 |
| L | CHEMICAL ABSTRACTS Band 109, Nr. 13, 16. September 1988, Seite 613, Spalte 1, Zusammenfassung Nr. 110323j, Columbus, Ohio, USA; J. SUWINSKI et al."Nitroimidazoles. VII. Replacement of nitro by amino group in 1,2-dimethyl-4,5-dinitroimidazole"; & ZESZ. NAUK. POLITECH. SLASK. CHEM., Nr. 111, 1986, Seiten 13-18 (Kat. X)<br>----- | 1-7 | |
| | | | **RECHERCHIERTE SACHGEBIETE (Int. Cl.4)** |
| | | | C 07 D 233/00<br>C 07 B 43/00 |

Der vorliegende Recherchenbericht wurde für alle Patentansprüche erstellt

| Recherchenort | Abschlußdatum der Recherche | Prüfer |
|---|---|---|
| BERLIN | 06-07-1989 | VAN AMSTERDAM L.J.P. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument